# EUROPEAN PATENT APPLICATION

(11) **EP 3 409 284 A2**
(43) Date of publication of application: **05.12.2018**
(21) Application number: 16731000.2
(22) Date of filing: 28.01.2016
(51) Int. Cl.: A61K 36/185, A61K 9/16, A61K 9/20, A61K 133/00, A61P 43/00

(54) **ABELMOSCHUS MANIHOT FLOWER PRODUCT**

(71) Applicant: Wang, Yalun, Shanghai 200235 (CN)
(72) Inventor: YANG, Surui, Shanghai 200235 (CN); LI, Jianbo, Shanghai 200235 (CN); LI, Wenyi, Shanghai 200235 (CN); DONG, Wenjia, Shanghai 200235 (CN); WANG, Jianshu, Shanghai 200235 (CN); WANG, Yalun, Shanghai 200235 (CN)
(74) Representative: Tergau & Walkenhorst Patentanwälte PartGmbB
(86) International application number: PCT/CN2016/072614
(87) International publication number: WO 2016/101932

(57) **Abstract**

The present invention relates to an Abelmoschus manihot flower product. The fresh flowers and dried flowers of Abelmoschus manihot crude drug contain special chemical components and have special biological activities. The goal of the present invention is to retain maximally and stabilize the ecological residue of the active substance in Abelmoschus manihot flowers. To that end, an optimal control point for material temperature and material humidity is obtained by mechanical and physical means. The key is to choose the Abelmoschus manihot flowers of which the pistils are in the ovaries. The bioflavonoid content in the product is high. The content of the marker substance, hyperoside is at least twice the amount of the standard of the pharmacopoeia. The product can be used for Chinese traditional medicinal material, food raw material product, or medicine, food, and cosmetic raw material.

## Description

### FIELD OF THE INVENTION

The invention belongs to the technical field of food development and relates to Abelmoschus manihot flower products.

### BACKGROUND OF THE INVENTION

The Abelmoschus manihot is a non-toxic plant, which is collected in the Dictionary of Medicinal Plant (1995, No. 4252, 4254, pp. 2075) published by Shanghai Science and Technology Press. The scientific name of the Abelmoschus manihot is Abelmoschus manihot (L.) Medicus, the corresponding code is 6000010003 in *Classification and codes of Chinese plants* (GB/T14467-93) which represents the national standard.

As a crude medicine, the fresh and dried Abelmoschus manihot flowers have specific chemical composition and biological activity. According to the report of the major project No. 20432030 of NSFC (Natural Science Foundation of China), department of natural medicine, Pharmaceutical Sciences School of Peking University disclosed that there are 23 active monomers in the Abelmoschus manihot flowers. Academy of Military Medical Sciences disclosed that there are 34 active health-related substances in the Abelmoschus manihot flowers, including 30 kinds of bioflavonoids. The Institute of Radiation Medicine, Academy of Military Medical Sciences, specially analyzed fat-soluble components of the Abelmoschus manihot flowers.

The Chinese PLA Medical Journal (Vol. 21, No. 5) reported that the Abelmoschus manihot flowers comprising 1.12% hyperoside.

Yongxiang Cao, the master of Xi'an Jiaotong Univeristy reported that a large amount of flavonoids existed in the Abelmoschus manihot. Most of the flavonoids are aglycon and glycosides with phenolic hydroxyl groups, which are active and may have a good prospect in anti-oxidation application. High content of the flavonoids in the Abelmoschus manihot is extremely unstable and has not been separated.

Since the Abelmoschus manihot flowers have the above-mentioned specific active substance, it has been sought for processing into products without losing or less losing its active ingredients. However, the products produced according to the report, literature and granted patent document in over 20 years comprised less and less active substances in the well-known raw materials, even less than 50% of the raw materials. By calculation, the flavonoid content in the product produced is only 20% of the conventional raw material in the patent No. 93110506.4; in the patent No. 201201082553.7 which is granted within 11 months from filing, the product of which has 5 kinds of flavonoids, only 17% of the raw material.

The products information of the Abelmoschus manihot flowers include material selection, dosage form, material temperature control in the preparation process, ovary selection of pistil are not disclosed in the corresponding reports.

### SUMMARY OF THE INVENTION

The objectives of the present invention is to most preserve the active substance in the Abelmoschus manihot flowers originally and maintain it stable. It is important to obtain the optimal control point for the material temperature and wetness and select the Abelmoschus manihot flowers comprising upwardly positioned pistil ovaries by mechanical and physical means. The products of the present invention have high flavonoid content, and the hyperoside content thereof is more than twice as the Pharmacopoeia criterion.

The present invention provides Abelmoschus manihot flower products, is characterized in that: selecting the Abelmoschus manihot flowers comprising upwardly positioned pistil ovaries as the raw material and processing them into dosage form; dehydrating the fresh Abelmoschus manihot flowers or moistening the dried Abelmoschus manihot flowers to a water content between 17% and 39% when preparing tablet-type products and granular products; controlling the temperature of the material with adequate water content to no more than 99.8°C, pressing the material to obtain tablet-like semi-finished products to be dried or extruding to obtain granular semi-finished products to be dried, and then drying the tablet-like material or the granular material under the temperature below 148°C, respectively, to obtain tablet-like products or granular products. When processing the flower slurry of the Abelmoschus manihot flowers comprising upwardly positioned pistil ovaries, adding water having three to five times the weight of the flower slurry to the flower slurry, and then spraying and centrifuging the material after passing through a colloid mill to obtain powdered products. During the whole process, the material is not exposed to acidic, alkaline or organic solvent substance, and no auxiliary materials are added.

The Abelmoschus manihot flower used as the raw material of the present invention, referred to as ovary flower, is characterized in that: the ovary flower is a flower bud or blooming flower having an ovary tissue in the center of the flower; the ovary tissue remains integrity, the lower part of the ovary is connected with the base tissue of stalk and the calyx which is organically integrated with the base tissue, the upper part of the ovary is organically connected with the stamen, and the circular base tissue of the ovary is organically connected with all petals; the ovary flower takes the ovary tissue of the Abelmoschus manihot flower as the elementary body, the ovary tissue of which is organically connected with the flower bud, petals, calyx and the base tissue of stalk, the pistil is upwardly positioned in the center of the ovary; the character of the term "ovary flower" is widely different with the corresponding term disclosed in the patent No. 93110506.4 which inventor is Ningning Xiong and other large quantities of literature.

The preparation method of the ovary flower is as follows: selecting the flower bud which pistil is not pollinated or fertilized or the blooming flower which pistil is partially pollinated and fertilized or the fresh flower which pistil is pollinated and fertilized no more than 10 hours after fertilization, and then dehydrating the flowers according to the known dehydration techniques.

The time of the tablet-like semi-finished products to be dried is no more than 2 hours, and the granular semi-finished products to be dried is no more than 1.5 hours, and the flower slurry to be powdered is no more than 58 mins.

The products of the present invention can be produced into Chinese medicine food or the raw materials of medicine, food, cosmetics.

In order to implementing the present invention, it is required to achieve the following elementary factors:
Except for the material temperature, moisture, raw material choosing, drying moisture and time, the equipment and process involved in the present invention adopt the conventional equipment and processing steps.

The material involved in the present invention referred to the material to be cut and ground to achieve the requirements of conventional tablets, granules and powders. The process and the character of the materials adopt the known techniques and conventional knowledge in the field. For example, although the preparation of the present invention does not propose any starch excipients for tablets, granules, etc., the present invention can achieve the effect of adding excipients in the conventional preparation, such as 16% polysaccharide gum contained in the prepared products keeps invariant when processed according to the present invention. The process and the requirements to the materials of preparing the products of the present invention are similar to that of preparing Xinhuang tablet by Xiamen traditional Chinese medicine Co., Ltd.

As for the definition of the Abelmoschus manihot flower, most of the Abelmoschus manihot flower involved in the disclosed documentation refers to the corolla part of the flower, whereas the corolla part refers to the petals and does not contain other tissues according to the common knowledge. The definition of the Abelmoschus manihot flower in the present invention guarantees the stability of the original active substance in the flower firstly, and secondly, the reproductive organs of plants are frequently the expression of their efficacy characteristics. As for flowers, the ovary and flower bud are the reproductive organs of flowers. If the preparation only adopts the corolla, the effects are not good enough. The purpose of the present invention to define the ovary flower further containing the stalk and calyx is to ensure that the ovary and flower bud are completed so as to guarantee the stability of the active substances; furthermore, if the flower ovary and bud are not picked, it is hard to estimate the efficiency and possibility in the practical flower picking process, and more importantly, the flower ovary and bud are definitely broken when picked, so that the probability of oxidation will increase, and key active substances will lose enormously.

The word "upwardly positioned" in the term "the pistil is upwardly positioned in the center of the ovary" is not an adjective and does not have the similar meaning to the word "sitting" in the libretto content like "I am sitting inside the embroidered room." The word "upwardly positioned" in the present invention represents shape and location. In the conventional flower products and flower Chinese traditional medicine, the selected pistil normally experiences a pretty long time from picking to fertilizing and is not positioned upwardly, which is different with the ovary flower in the present invention. When selecting ovary flowers, the flower buds of the blooming flowers, although the stamens, the pistil and ovary are completed, are not fertilized, and the stamen pollen has not yet popped out of the holes. The fresh flower after the stamen pollen popped out refers to the ovary flower which pistil is pollinated and fertilized no more than 10 hours from picking to fertilizing. Due to different conditions such as weather, seasons, regions, seeds and growing conditions, the popping time of different stamen pollen are different. The determination method of deciding the popping time is to direct observing the flowers with a high magnification magnifier to determine when blooming. After the qualified fresh flowers are picked, known techniques are applied to prepare the dried flowers, such as the technical scheme disclosed in the patent No. 93110506.4 which patentee is Li Wang. No matter the drawings in the patent No. 93110506.4 which inventor is Ningning Xiong or the pictures disclosed in the documentation corresponding to the Abelmoschus manihot flower, the pictures are all focus on the corolla. The Contemporary Chinese Dictionary explains the term "corolla" (in Chinese phonetic letters as "Huaguan") in page 538 as "a part of the flower, which containing a plurality of petals". The Concise Dictionary of Biology explains the term "corolla" in page 611 as the general term of petals. Obviously, the Abelmoschus manihot flower disclosed in the prior art refers to the corolla, that is to say, refers to the petals without other tissues, the ovary and upwardly positioned pistil ovary are not mentioned, it is therefore the fact that the last paragraphs of the background art are appeared.

The limitation of being exposed to acidic, alkaline or organic solvent substances also includes the internal and external environments except for the equipment and water.

There is no lower limit for the data in the present invention only because the upper limit data affects the material character, that is, the upper limit data is the threshold of keeping the material character in the product flow unchanged, and below this upper limit, no material character change happened just a difference in production efficiency. For example, polysaccharide gum in the material may go variation and decomposition instantaneous under certain moisture when the temperature exceeds 99.8°C. As for how to control the dryness and wetness of the present invention belongs to the common knowledge, this is because the dryness and wetness are controlled by the temperature. Under this circumstance, the material is cooled down with the evaporation of water. In the microscopic molecular part of the material, as the water evaporates, the conditions for the variation and decomposition do not exist due to the temperature decreased.

### DETAILED DESCRIPTION OF THE EMBODIMENTS

### Embodiment 1:

Selecting the dried flower buds of the ovary flowers of the Abelmoschus manihot flowers 30kg, and crushing them into pieces having a size less than 1mm*1.1mm and a size less than 1mm*1.3mm by hammer crusher and then mixing them; the obtained material is 29.7kg after weighting, the difference is the material consumption; the material consumption value is large due to processing in a small batch; the material contains 8% water (by weight percentage); calculating by the water content of the material below 38%, the equations are: 29.7kg minus the water content, 38% minus 8% equals 30%, then multiplied by 29.7kg equals 0.89kg, this result means that the material needs to increase the water content; then heating the water to the temperature of 98°C, and moistening the material with water having the temperature of 98°C by spray humidification method, and then extruding the moistened material by a micro granulator with screw to obtain semi-finished products to be dried; and then drying the semi-finished products in the small drying oven under the temperature of 110°C for 23 mins to obtain granular products.

### Embodiment 2:

Selecting the dried ovary flowers of the Abelmoschus manihot flowers 30kg, and crushing them into pieces having a size less than 1mm*0.5mm and a size less than 1mm*1.6mm by hammer crusher and then mixing them; calculating and then preparing the moistened material according to the embodiment 1; and then pressing the material to obtain tablet-like semi-finished products to be dried by using the equipments and process of a pharmaceutical factory of Guanzhuang City of China; and drying the semi-finished products by a rolling-chain type drying machine for 18 mins to obtain tablet-like products.

### Embodiment 3:

Millet Institute of Hebei Institute of Agricultural Sciences provides people skilled in the art and a set of equipment of spraying, drying, colloid milling and filtering the slurry to prepare the products of the present invention. The preparation steps are as follows:
Selecting the mixture of fresh flower buds and the blooming ovary flowers of the Abelmoschus manihot flowers 300kg, processing them into slurry by a blade-type beater, and then processing the slurry into the powdered products.

## Claims

1. Abelmoschus manihot flower products, which is **characterized in that**: selecting the Abelmoschus manihot flowers comprising upwardly positioned pistil ovaries as the raw material and processing them into dosage form; dehydrating the fresh Abelmoschus manihot flowers or moistening the dried Abelmoschus manihot flowers to a water content between 17% and 39% when preparing tablet-type products and granular products; controlling the temperature of the material with adequate water content to no more than 99.8°C, pressing the material to obtain tablet-like semi-finished products to be dried or extruding to obtain granular semi-finished products to be dried, and then drying the tablet-like material or the granular material under the temperature below 148°C, respectively, to obtain tablet-like products or granular products; when processing the flower slurry of the Abelmoschus manihot flowers comprising upwardly positioned pistil ovaries, adding water having three to five times the weight of the flower slurry to the flower slurry, and then spraying and centrifuging the material after passing through a colloid mill to obtain powdered products; during the whole process, the material to be prepared is not exposed to acidic, alkaline or organic solvent substances, and no auxiliary materials are added into the materials.

2. The Abelmoschus manihot flower products according to claim 1, the Abelmoschus manihot flower used as the raw material, referred to as ovary flower, is **characterized in that**: the ovary flower is a flower bud or blooming flower having an ovary tissue in the center of the flower; the ovary tissue remains integrity, the lower part of the ovary is connected with the base tissue of stalk and the calyx which is organically integrated with the base tissue, the upper part of the ovary is organically connected with the stamen, and the circular base tissue of the ovary is organically connected with all petals; the ovary flower takes the ovary tissue of the Abelmoschus manihot flower as the elementary body, the ovary tissue of which is organically connected with the flower bud, petals, calyx and the base tissue of stalk, the pistil is upwardly positioned in the center of the ovary; the character of the term "ovary flower" is different with the corresponding term disclosed in the patent No. 93110506.4 which inventor is Ningning Xiong and other large quantities of literature.

3. The preparation method of the ovary flower of the Abelmoschus manihot flower according to claim 1, which is **characterized in that**: selecting the flower bud which pistil is not pollinated or fertilized or the blooming flower which pistil is partially pollinated and fertilized or the fresh flower which pistil is pollinated and fertilized no more than 10 hours after fertilization, and then dehydrating the flowers according to the known dehydration techniques.

4. The products according to claim 1, which is **characterized in that**: the time of the tablet-like semi-finished products to be dried is no more than 2 hours, and the granular semi-finished products to be dried is no more than 1.5 hours, and the flower slurry to be powdered is no more than 58 mins.

5. The products according to claim 1, which is **characterized in that**: the products can be produced into Chinese medicine food or the raw materials of medicine, food, cosmetics.
